# EUROPEAN PATENT SPECIFICATION

(11) **EP 3 417 268 B1**
(45) Date of publication and mention of the grant of the patent: **01.04.2020**
(21) Application number: 17707210.5
(22) Date of filing: 20.02.2017
(51) Int. Cl.: G01N 3/42, G01N 33/00

(54) **METHOD TO OPTIMIZE THE STORAGE OF HARVESTED SUGAR BEETS**
VERFAHREN ZUR OPTIMIERUNG DER LAGERUNG VON GEERNTETEN ZUCKERRÜBEN
PROCÉDÉ D'OPTIMISATION DE LA CONSERVATION DES BETTERAVES SUCRIÈRES APRÈS RÉCOLTE

(30) Priority: 18.03.2016 BE 201605198; 27.09.2016 EP 16190802
(43) Date of publication of application: 26.12.2018
(73) Proprietor: SESVanderHave N.V., 3300 Tienen (BE)
(72) Inventor: HENRY, Nicolas, 3300 Tienen (FR); SUIVENG, Francois, 3300 Tienen (FR); TOSSENS, Alain, 3300 Tienen (BE); HOREMANS, Stefaan, 3300 Tienen (BE); TSCHOEP, Hendrik, 3300 Tienen (BE)
(86) International application number: PCT/EP2017/053730
(87) International publication number: WO 2017/157612

(56) References cited:
- A.G. Bengough ET AL: "Mechanical impedance to root growth: a review of experimental techniques and root growth responses", Journalof Soil Science, 1 January 1990 (1990-01-01), pages 341-358, XP055299863, Retrieved from the Internet: URL:http://www.plantstress.com/methods/Roo t Methods PORTAL/Mechanical impedance.pdf [retrieved on 2016-09-05]
- PAN LEIQING ET AL: "Predict Compositions and Mechanical Properties of Sugar Beet Using Hyperspectral Scattering", FOOD AND BIOPROCESS TECHNOLOGY, SPRINGER NEW YORK, NEW YORK, NY, vol. 9, no. 7, 7 March 2016 (2016-03-07), pages 1177-1186, XP035950831, ISSN: 1935-5130, DOI: 10.1007/S11947-016-1710-5 [retrieved on 2016-03-07]

## Description

### Field of the invention

The present invention is in the field of the sugar beet industry and of agronomy. More particularly, the present invention discloses a method for determining the storage capacity of a sugar beet.

### Background of the invention

Sugar beet is an important agricultural crop in temperate and subtropical regions.

For increasing the competitiveness of the sugar beet crop, sugar refineries need the possibility to store sugar beets for an extended period after harvest.

Indeed, the harvesting period cannot be postponed too much, since sugar beets should be harvested before the occurrence of permanent sub-zero temperatures and before too heavy rain. Thus sugar beets are harvested at a given period (e.g. in October-November) and stored with the assumption that the temperatures are not too high, but that no freezing-thawing of the harvested roots occurs. In practice this risk is minimized using special types of tissue to cover the storage piles. However, such measures can only buffer against a limited range of extreme temperatures. Moreover, mechanical damages during harvest and root rot during harvest or occurring during the growth phase of the sugar beet impair the conservation capacities of the harvested sugar beet roots. One main associated problem to mechanical damages to roots and/or to the presence of molds, is the loss of sucrose upon storage of the harvested beets. Beside a reduction of the sucrose content, where even a small percentage of reduction is, in practice, a considerable loss in view of all the fixed costs, any metabolic activity, either endogenous or secreted by the molds, results into increased amount of different organic compounds (e.g. non-sucrose sugars such as dextranes, organic acids) and invert sugars that negatively affect the extraction efficiency of sucrose in the sugar refinery. More into details, in the few days after harvest, sucrose losses mainly occur due to the metabolism of the sugar beet (e.g. wound healing and respiration), thereafter endogenous metabolic activity declines, preserving the remaining sucrose. However, a second source of sucrose losses, due to molds (bacteria or fungi), but also nematodes, insects,... becomes a major factor upon prolonged storage. For instance, under the current practice, the losses are shown to increase exponentially after 270 °C^{∗}days of storage (measured on the outside temperature; i.e. about 300-350 degrees day when the temperature is measured inside a pile of sugar beet; the value is computed as the sum of the (maximal/2+minimal/2) temperature of a day (if positive; otherwise zero) for all the days of storage).

Thus prolonged storage is not yet a valuable option, and this forces sugar refineries to work continuously in order to process the sugar beets as fast as possible (e.g. not to pass too much the 270°C^{∗}days limit).

In other words, upon current practice, the loss of sucrose, and consequently the increased level of contaminant products that have an aversive effect on sucrose extraction, can be reduced by storing the sugar beet for shorter time, but at the expense of huge capacities used only for a few weeks per year.

There have been some attempts to classify sugar beet on their adaptation to long-term storage and also to improve varieties for this property.

However, these attempts have been, for a large part, unsuccessful and imprecise. For instance a process for improving varieties requires to grow, harvest and store sugar beets in well-controlled conditions, then to measure several times the remaining sucrose content. This is a rather complex, costly and time-consuming approach.

Therefore, there is a need to improve the existing techniques.

### Summary of the invention

The present invention discloses a method for predicting sugar beet resistance to (biological) factors causing root damages and/or degradation upon storage and/or to factors causing sucrose loss upon storage, comprising to quantify the mechanical resistance of the root of the said sugar beet.

A related aspect of the present invention is a method for predicting sugar beet resistance to biological factors causing damages to the root during culture, comprising to quantify the mechanical resistance of the root of sugar beets from the same genetic origin.

In these methods, preferably, the quantification of the mechanical resistance is carried out by penetrometry.

In these methods, preferably or alternatively, the quantification of the mechanical resistance is carried out upon recording the force (kg/cm²) needed to push a probe of a given section through the outer layer of the root, more preferably, wherein the recorded force is the maximal force (kg/cm²) needed to push the probe of a given section through the root.

Advantageously, the method of the present invention allows that the sugar beets from several lots are stratified in function of the mechanical resistance of the root of the said sugar beets (the quantification, if invasive, can advantageously be performed on a few, e.g. 8, roots from any one of the lots and is representative on the overall resistance of the lot, allowing to stratify lots still comprising a large number of undamaged roots) . In this method, advantageously, the less resistant sugar beet lots are processed firstly by sugar refineries.

Alternatively (or in addition), in this method, the less resistant sugar beet lots are treated (i.e. controlled temperature or humidity, addition of chemicals) for reducing sucrose losses.

In the present method, the biological factors leading to root damages are sugar beet pathogens and/or parasites, possibly selected from the group consisting of bacteria, fungi, nematodes and larvae thereof, arthropods and larvae thereof, insects (e.g. aphid or root maggot fly) and larvae thereof.

Still another related aspect of the present invention is the use of a penetrometer for sorting sugar beets according to their resistance to sucrose loss upon storage.

### Detailed description of the invention

The inventors have firstly considered that the density, marc content and/or the resistance of the inner layers of sugar beet are associated to resistance towards loss of sugar upon storage. Indeed, more dense tissues, especially the presence of tissues rich in sucrose that are further protected by more dense fibers are believed to be more resistant to degradation.

However, the density and/or resistance of the inner layers of a sugar beet does not correlate with its resistance to sucrose loss.

In fact, any general method for the prediction of the resistance of sugar beets upon storage (loss of sucrose; resistance to root rot) is facing towards two major hurdles: the intra lot variability and the variability caused by the field conditions.

The inventors have nevertheless succeeded in developing a more complex measurement method (specifically) based on the measure of the mechanical resistance of the sugar beet; for instance through the force to push a probe through the outer layer of the root (penetrometry). This method requires additional mechanical and electronical devices to precisely control the force to be applied and to specifically measure the maximal force that has been needed to push the probe through the outer layer of the sugar beet. In addition, the limited thickness of the outer layer of the root implies that the system must be very well calibrated and controlled. Also the time period between harvest and measurement can have a major impact on penetrometry measurements. Finally, the heterogeneity of the beet root and thus the consequences on the validity and/or on the relevance of the generated measures are major concerns explaining why the method of the present invention has not yet been developed and thus proposed at an industrial scale.

The inventors have shown that the variability between one harvested lot or between sugar beets from the same origin grown in different field conditions is limited by comparison to the variability caused by the genetic origin of the sugar beet. The inventors have further shown that the sugar beet (outer layer) is homogenous enough so as to require only one measurement per beet. To summarize, despite all the above-prejudices, the inventors have observed very good correlations between the penetrometry measurements and the resistance towards sugar loss and/or towards biological factors causing damages (and/or stress) to the roots of the sugar beet.

Therefore, a first aspect of the present invention is a method to sort sugar beets in function of their resistance to sucrose loss upon storage, comprising to (specifically) measure the mechanical resistance of the sugar beet root. Preferably, the mechanical resistance is the measure (quantification) of the (maximal) force (kg/cm²) needed to push a probe (of a given section) through the root of the sugar beet. In practice, the maximal force is recorded, and is advantageously considered in the present invention to reflect the resistance of the outer layer of the root towards damages. This method allows for instance to process firstly the non-resistant harvested sugar beets (requiring a low force to push the probe), then the sugar beets predicted to be more resistant, having in mind the goal to minimize the overall sucrose loss, or to select lots with very low sucrose losses, and thus accumulation of reduced levels of by-products such as dextranes or invert sugar that negatively affect the sucrose extraction efficiency, so as to allow easier downstream processes. Alternatively, this method allows to limit the treatments of harvested sugar beets against root rot and/or loss of sucrose only to the lots determined as sensitive, or to escalate the treatments in function of the predicted sensitivity. Biological factors causing biological stress to the roots of sugar beet preferably refer to (living) bacteria, (living) fungi, such as fungal roots (e.g. *Rhizoctonia solani*), (living) nematodes (e.g. Ditylenchus), (living) insects and larvae thereof (e.g. fly larvae causing root maggot), (living) arthropods and larvae thereof.

In the present invention, fungi and bacteria are sometimes collectively mentioned as molds.

Alternatively, or in addition, root rot comprises root damages during harvest, and root disease that occur during the growth phase, such as fungal rots (e.g. *Rhizoctonia solani*).

Preferably, the factors causing biological stress to the roots of sugar beet are those attacking the harvested sugar beet, thus resulting into sucrose loss (as well as into increased level of organic metabolites such as non-sucrose sugars and organic acids).

Still another related aspect of the present invention is a device adapted for the specific measurement (quantification) of the force (kg/cm²) needed to push a probe of a given section through the root of a sugar beet, comprising a probe of a given section, a system to apply a defined force to the probe and means to record the force (kg/cm²) that has been needed to push the probe through the outer layer of the sugar beet, and the corresponding use thereof.

The section of the probe is not particularly crucial. The probe can be a rod and/or in the form of a cylinder, although rectangular sections can also be used. Typical sections range between 0.1 cm² and 1 cm² (such as about 0.35 cm²).

Good results have been obtained with devices commercialized under the brand name PENEFEL (Setop, Cavaillon, France).

In the methods of the present invention, the maximal force that has been required for the insertion of the probe into the root of a sugar beet is recorded. In practice, the measure (any measure) is performed on the roots of at least 8 beets (possibly on at least 20 beets) from a same (genetic) origin, as the inventors have shown that the intra-lot variability is efficiently taken into account with this limited number of measures. Sometimes, the measure is taken at three different positions of one root.

### Brief description of the figures

Figure 1. Effect of the number of roots.
Figure 2. Penetrometry values in function of the Rot index (Panel A), or of the loss of sucrose (Panel B).
Figure 3. Correlation between the mechanical resistance and the outer layer content of hemicellulose (Panel A) and of cellulose (Panel B).

### Examples

### Comparative example

The inventors have compared the marc content (reflecting the fiber content surrounding the sucrose-containing tissues) of the sugar beet and the rot index (the protocol is detailed in the Example 2) or the loss of sucrose for series of harvested sugar beet varieties.

No correlation can be established between the resistance or the density of sugar-containing layers of sugar beet and the rot index or the loss of sucrose.

### Example 1

### General validation of the system

The inventors have firstly compared penetrometry measurements (PENEFEL penetrometer commercialized by Setop company, Cavaillon, France) performed at different places of a single sugar beet and measured no real difference. As a precautionary measure, the inventors nevertheless propose to establish the measures in the same region of the roots (e.g. at the maximal diameter of the root) and/or to perform several measurements (e.g. at least three) for any root.

Then the inventors have compared the measurements on a same lot, but taken at different times after harvest.

Again, no real difference has been measured, though the inventors consider that it is preferable to perform measurements on roots after a constant period after harvest.

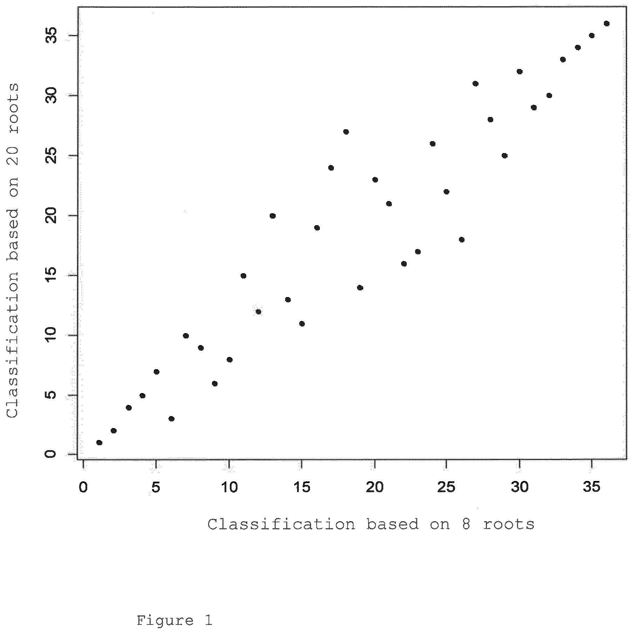

Then the inventors have measured the minimal number of beets to take into account for the intra lot variability (same field conditions, same genetic background). The inventors have noticed a minor variability. When performing the measurement on 8 beets, the variability becomes much lower than the variability caused by different genetic origin (see below), and the correlation between the measurements on 8 and on 20 different roots from one lot is excellent; see Figure 1.

### Example 2.

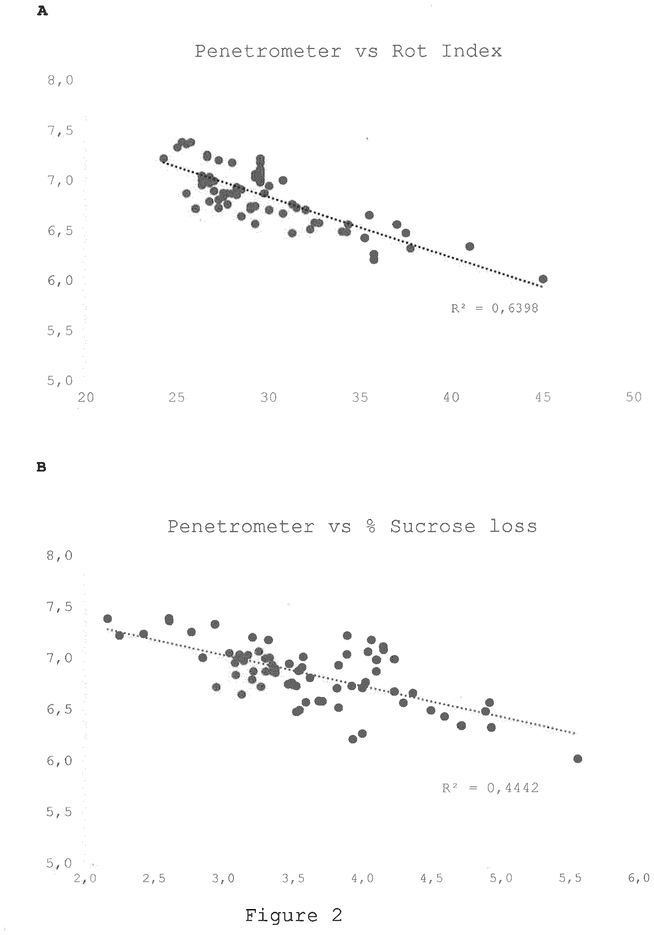

Having validated the system, the inventors then compared the penetrometry data and the rot index or the loss of sugar for series of harvested sugar beet varieties (Figure 2).

The maximal force for the insertion of the probe is measured on the basis of three measures per sugar beet root, and of 20 roots per condition. The detection limit is, in this example, of 2kg/cm². A good correlation can be established between the resistance of the beet root and either the rot index or the loss of sucrose (the inter lot variance is 10 or even 50 times bigger than the intra lot variance). The less resistant beets have lost almost twice the amount of sucrose than the most resistant ones. More into details, between 60 and 75 manually harvested sugar beet roots from the same lot (genetic origin and, in these settings, field conditions) are separated for three different analysis: the measure of the sugar content at the harvesting time; the conservation analysis and the measure by penetrometry. The conservation analysis is carried out after causing a controlled mechanical damage to the manually-harvested roots, aiming at mimicking, in a more homogenous fashion, the damages caused by mechanical harvest, then conservation is done at 12°C for 7 days, then at 8°C for 44 days (436°C^{∗}days) with the humidity controlled at 95%, for mimicking the weather conditions during autumn in North-Western Europe.

Then the sucrose content and the root rot index are measured. The root rot index is assessed on the basis of 5 classes: Class 1; healthy beets; Class 2, beets with up to 25% of the surface subjected to rot; Class 3, beets with between 25% and 50% of the surface subjected to rot; Class 4, beets with between 50% and 75% of the surface subjected to rot; Class 5, rotten beets. The index is the averaged sum. The inventors have noticed good homogeneity between lots.

### Example 3

The inventors have then compared in the field the rot index and the insect damages for series of sugar beet varieties.

Again, there is a negative correlation between these damages and the resistance the root of the sugar beet of the same origin as measured by penetrometry.

### Example 4

The inventors have then developed sugar beets on the basis of the resistance of their roots to the penetrometer. The inventors have stratified sugar beets from the different genetic origins in ten classes according to the measured resistance to the penetrometer of 20 roots per genetic origin. The sugar beets of different origins that have been attributed to the most resistant class have been crossed so as to obtain a progeny with an even more marked resistance phenotype.

### Example 5

### Alternative measurements of the mechanical resistance: chemical composition of the external layer

The inventors have then analyzed the external layer (peel) of sugar beets to determine if their chemical content presents variations and if these variations are correlated either to the direct methods for measuring the mechanical resistance of the outer layer of the sugar beet (as disclosed above), and/or if there is a correlation with the sucrose loss or with the resistance to biological factors during culture.

To do so, the external peels have been obtained from several sugar beet lots (in practice at least the peels from 4 sugar beets per condition).

Then, the peels have been mechanically homogenized (step 1) and their soluble contents (e.g. sugars) are removed with hot water (step 2). A neutral detergent solution is then added on the remaining marc, so as to remove the pectin and the proteins (step 3) . Then a first acid solution is added, so as to degrade the hemicellulose (step 4), then a second acid solution (70% H2S04) is added to degrade the cellulose (step 5), before ashing at 550°C so as to remove the lignin component and to end with the remaining ashes (step 6). The solid content of the compositions is measured at every step, and the (absolute or relative) abundance of one key component (e.g. cellulose or hemicellulose) is determined by difference (e.g. the hemicellulose content is deduced by subtracting to the solid content after step 3 the solid content after step 4).

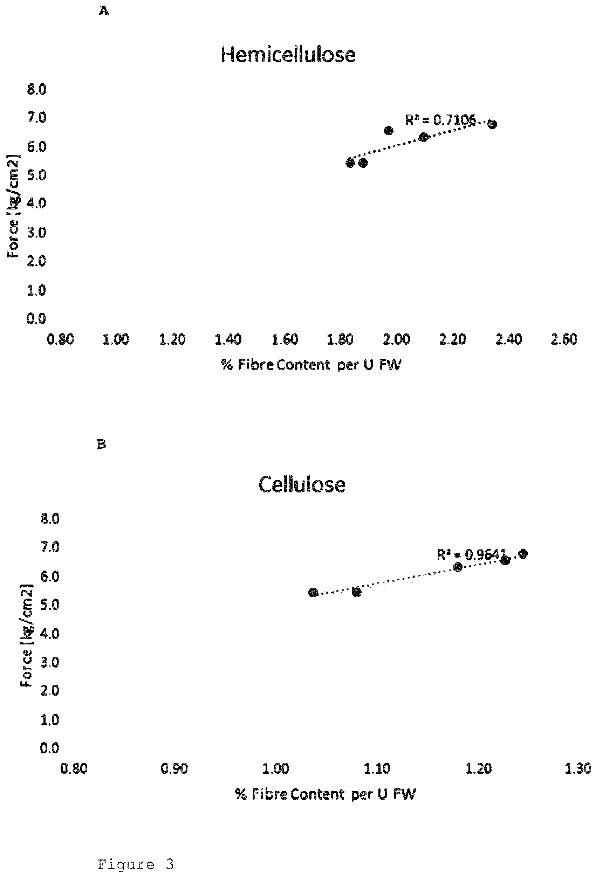

From these results, the inventors have noticed very good correlations between the hemicellulose (and/or the cellulose) content and the penetrometer measurements (Figure 3) and/or with the resistance to sucrose loss and/or with the resistance to biological factors during culture. The lignin content, on the other hand, is more constant between lots, and thus a poor readout of the mechanical resistance (R² coefficient of about 0.4).

As a second alternative, homogenized peels (step 1 as above or more preferably, from step 3 as above, after removal of pectin) are analyzed by near-infra red measurements so as to determine their content in hemicellulose and cellulose (the measurement is mainly for =CO and -COH residues, abundant in these polymers). Again, the inventors have noticed very good correlations between the hemicellulose (and/or the cellulose) content(s) and the penetrometer measurements and/or with the resistance to sucrose loss and/or with the resistance to biological factors during culture.

In other words, these methods are applied to the external structure of the sugar beet and reflect the mechanical resistance of the sugar beet.

## Claims

1. A method for predicting sugar beet resistance to factors causing root damages and/or to factors causing sucrose loss upon storage, comprising to quantify the mechanical resistance of the root of the said sugar beet.

2. A method for predicting sugar beet resistance to biological factors causing root damages during culture, comprising to quantify the mechanical resistance of the root of sugar beets from the same genetic origin.

3. The method of claim 1 or 2, wherein the quantification is carried out by penetrometry.

4. The method according to any one of the preceding claims 1 to 3, wherein the quantification is carried out upon recording the force (kg/cm²) needed to push a probe of a given section through the outer layer of the root.

5. The method according to any one of the preceding claims 1 to 4, wherein the quantification is the maximal force (kg/cm²) needed to push the probe of a given section through the root.

6. The method according to any one of the preceding claims 1 to 5, wherein the sugar beets from several origins are stratified in function of the mechanical resistance of the root of the sugar beets and/or in function of the maximal force needed to push the probe through the root.

7. The method of claim 6, wherein the less resistant sugar beet lots are processed firstly by sugar refineries.

8. The method of claim 6 or 7, wherein the less resistant sugar beet lots are treated for reducing sucrose losses.

9. The method according to any one of the preceding claims, wherein the (biological) factors leading to root damages are sugar beet pathogens and/or parasites, preferably selected from the group consisting of bacteria, fungi, nematodes and larvae thereof, arthropods and larvae thereof, insects and larvae thereof.

10. Use of a penetrometer for sorting sugar beets according to their resistance to sucrose loss upon storage.

## Patentansprüche

1. Verfahren zur Vorhersage der Resistenz von Zuckerrüben gegen Faktoren, die Wurzelschäden verursachen, und / oder gegen Faktoren, die Saccharoseverluste bei der Lagerung verursachen, umfassend die Quantifizierung der mechanischen Resistenz der Wurzel der Zuckerrüben.

2. Ein Verfahren zur Vorhersage der Resistenz von Zuckerrüben gegen biologische Faktoren, die während der Kultur Wurzelschäden verursachen, umfassend die Quantifizierung der mechanischen Resistenz der Wurzel von Zuckerrüben gleichen genetischen Ursprungs.

3. Verfahren nach Anspruch 1 oder 2, wobei die Quantifizierung durch Penetrometrie erfolgt.

4. Verfahren nach einem der vorhergehenden Ansprüche 1 bis 3, wobei die Quantifizierung beim Aufzeichnen der Kraft (kg / cm²) durchgeführt wird, die erforderlich ist, um eine Sonde eines gegebenen Abschnitts durch die äußere Schicht der Wurzel zu drücken.

5. Verfahren nach einem der vorhergehenden Ansprüche 1 bis 4, wobei die Quantifizierung die maximale Kraft (kg / cm²) ist, die benötigt wird, um die Sonde eines gegebenen Abschnitts durch die Wurzel zu drücken.

6. Verfahren nach einem der vorhergehenden Ansprüche 1 bis 5, wobei die Zuckerrüben verschiedener Herkunft in Abhängigkeit geschichtet werden von dem mechanischen Widerstand der Wurzel der Zuckerrüben, und / oder in Abhängigkeit von der maximalen Kraft, die zum Drücken der Sonde benötigt wird, durch die Wurzel.

7. Verfahren nach Anspruch 6, wobei die weniger widerstandsfähigen Zuckerrübenchargen zuerst von Zuckerraffinerien verarbeitet werden.

8. Verfahren nach Anspruch 6 oder 7, wobei die weniger widerstandsfähigen Zuckerrübenchargen behandelt werden, um Saccharoseverluste zu verringern.

9. Verfahren nach einem der vorhergehenden Ansprüche, wobei die (biologischen) Faktoren, die zu Wurzelschäden führen, Zuckerrübenpathogene und / oder Parasiten sind, vorzugsweise ausgewählt aus der Gruppe bestehend aus Bakterien, Pilzen, Nematoden und Larven davon, Arthropoden und Larven davon, Insekten und Larven davon.

10. Verwendung eines Penetrometers zum Sortieren von Zuckerrüben nach ihrer Beständigkeit gegen Saccharoseverlust bei Lagerung.

## Revendications

1. Une méthode pour la prédiction de la résistance d'une betterave à des facteurs provoquant des dommages aux racines et/ou à des facteurs provoquant la perte de saccharose au cours de l'entreposage, comprenant la quantification de la résistance mécanique de la racine de ladite betterave.

2. Une méthode pour la prédiction de la résistance d'une betterave à des facteurs provoquant des dommages aux racines au cours de la culture, comprenant la quantification de la résistance mécanique de la racine de betteraves de la même origine génétique.

3. La méthode de la revendication 1 ou 2, dans laquelle la quantification est réalisée par pénètrométrie.

4. La méthode selon une quelconque des revendications 1 à 3, dans laquelle la quantification est réalisée en mesurant la force (kg/cm²) nécessaire à l'introduction d'une sonde d'une section donnée à travers la couche externe de la racine.

5. La méthode selon une quelconque des revendications 1 à 4, dans laquelle la quantification est la force maximale (kg/cm²) nécessaire à l'introduction de la sonde d'une section donnée à travers la racine.

6. La méthode selon une quelconque des revendications 1 à 5, dans laquelle les betteraves de différentes origines sont stratifiées en fonction de la résistance mécanique de la racine des betteraves et/ou en fonction de la force maximale nécessaire à l'introduction de la sonde à travers la racine.

7. La méthode de la revendication 6, dans laquelle les lots de betteraves les moins résistants sont traités en priorité par les raffineries de sucre.

8. La méthode de la revendication 6 ou 7, dans laquelle les lots de betteraves les moins résistants sont traités pour réduire les pertes en saccharose.

9. La méthode selon une quelconque des revendications précédentes, dans laquelle les facteurs (biologiques) conduisant à des dommages aux racines sont des pathogènes et/ou des parasites de la betterave, de préférence choisis dans le groupe constitué de bactéries, de champignons, de nématodes et de leurs larves, d'arthropodes et de leurs larves, d'insectes et de leurs larves.

10. Utilisation d'un pénétromètre pour le tri des betteraves en fonction de leur résistance à la perte de saccharose au cours de l'entreposage.
